# EUROPEAN PATENT APPLICATION

(11) **EP 1 136 557 A1**
(43) Date of publication of application: **26.09.2001**
(21) Application number: 00201045.2
(22) Date of filing: 21.03.2000
(51) Int. Cl.: C12N 15/31, C07K 14/285, A61K 39/102

(54) **Proteins and polypeptides from Haemophilus influenzae involved in paracytosis, nucleotide sequences encoding them and uses thereof**

(71) Applicant: DE STAAT DER NEDERLANDEN VERTEGENWOORDIGD DOOR DE MINISTER VAN WELZIJN, VOLKSGEZONDHEID EN CULTUUR, NL-2280 HK Rijswijk (NL)
(72) Inventor: Van den Schilfgaarde, 3720 BE Bilthoven (NL); Van Alphen, Aloysius Joannes Wilhelmus, 3720 BE Bilthoven (NL)
(74) Representative: Jorritsma, Ruurd

(57) **Abstract**

The present invention relates to proteins and polypeptides that are involved in paracytosis, and to nucleotide sequences that encode such proteins or polypeptides. In particular, the present invention relates to proteins and polypeptides that are involved in paracytosis through epithelial cell layers such as lung epithelial cell layers, and to nucleotide sequences that encode such proteins or polypeptides. More specific, the (nucleotide sequences encoding such) proteins or polypeptides have been derived from the micro-organism *Haemophilus influenzae*, being the nucleotide sequence identified as ORF HI 0636 (SEQ ID NO 1) and/or ORF HI 0638 (SEQ ID NO 2) and/or mutants, variants, analogues, parts and/or fragments and/or natural homologues thereof.

The invention also relates to methods for the preparation of such proteins or polypeptides, to genetic constructs containing these nucleotide sequences, and to some specific uses of these proteins/polypeptides, nucleotide sequences and/or genetic constructs.

## Description

Present invention relates to proteins and polypeptides that are involved in paracytosis, and to nucleotide sequences that encode such proteins or polypeptides.

In particular, the present invention relates to proteins and polypeptides that are involved in paracytosis through epithelial cell layers such as lung epithelial cell layers, and to nucleotide sequences that encode such proteins or polypeptides.

More in particular, the present invention relates to (nucleotide sequences encoding) such proteins or polypeptides that have been derived from the micro-organism *Haemophilus influenzae.*

The invention also relates to methods for the preparation of such proteins or polypeptides, to genetic constructs containing these nucleotide sequences, and to some specific uses of these proteins/polypeptides, nucleotide sequences and/or genetic constructs.

Other aspects and embodiments of the invention will become clear from the description given hereinbelow.

The passage of micro-organisms inbetween cells - e.g. inbetween cells of tissues and/or of cell layers such as the epithelial cells of the respiratory tract - is called paracytosis.

Generally, such paracytosis involves a change in the intercellular permeability of the tissue or cell layer, which is caused by the micro-organism and/or by a factor produced by the micro-organism.

For instance, various intestinal pathogens perturb the paracellular barrier by production of toxins, such as the zonula occludens toxin (ZOT) and the hemagglutin/ protease of *Vibrio cholerae*, toxin A and toxin B of *Clostridium difficile*, and VacA of *Helicobacter pylori.* However, such toxins may exert a pathologic effect on epithelial cells, and thus generally are not suited for therapeutic and/or pharmaceutical use.

Some other bacteria that employ paracytosis or interjuntional passage are for instance the spirochetes *Borrelia burgdorferi* and *Treponema pallidum.* The mechanisms underlying this passage are unknown.

The Gram-negative bacterium *Haemophilus influenzae* is a natural inhabitant of the human upper respiratory tract and an important pathogen responsible for respiratory tract infections as well as systemic disease. Encapsulated strains with a serotype b polysaccharide cause invasive *H. influenzae* disease such as meningitis. Infections by nonencapsulated (nontypeable) *H*. *influenzae* are mostly limited to respiratory mucosal surfaces and result from contiguous spread of *H*. *influenzae* from the nasopharynx. Nontypeable *H*. *influenzae* is a frequent cause of otitis media, and of lower respiratory tract infections, particularly in patients with chronic obstructive pulmonary disease (COPD) and cystic fibrosis (CF).

The first interaction of *H*. *influenzae* with its host is an interaction between the bacterium and the epithelial cell layer of the respiratory tract. *H influenzae* is generally considered to be an invasive organism, since in the onset of systemic disease as well as during persistent lower respiratory tract infections *H*. *influenzae* bacteria pass through the respiratory tract epithelium.

It has been suggested in the art that this passage between the epithelial cells, which is characteristic for *H. influenzae,* involves selective disclosure of intercellular junctions (van Schilfgaarde et al., Infect.Immun. 63:4729-4737 (1995)). However, the mechanisms by which *H. influenzae* is able to effect disclosure of these junctions, as well as the factors involved therein, are as yet unknown.

In the invention, it has now been found that the genome of *H*. *influenzae,* and in particular of *H. influenzae* strain A960053, contains two open reading frames ("ORFs") that encode factors involved in paracytosis. These ORFs - which have the nucleotide sequences given in SEQ ID NO.1 and SEQ ID NO.2 - are also referred to hereinbelow as "ORF HI0636"or "*HI0636*"; and "ORF HI0638" or "*HI0638*", respectively. Clones of *E.coli* DH5α containing ORF HI0636 and/or ORF HI0638 showed significant increase in penetration of epithelial cell layers of the human bronchial epithelial cell line NCI-H292

*HI0636* and *HI0638* each encode a small protein involved in paracytosis, which proteins are referred to hereinbelow as "HI0636" and "HI0638" respectively.

In the invention, these proteins (as well as the nucleotide sequences encoding them) may for instance be used to alter the permeability of tissues or cell layers, and in particular of epithelial cell layers such as lung epithelial cell layers. Thus, the proteins and/or nucleotide sequences of the invention may find application in the pharmaceutical field, for instance in the specific applications mentioned hereinbelow.

It is also envisaged that knowledge of the process of invasion of *H*. *influenzae* through the epithelium and of the factors involved therein as provided by the present invention will be of importance in developing factors or compounds that may be used to block paracytosis as well as to obtain insight into the invasion mechanisms used by various bacteria. Furthermore, the identification of these bacterial components involved in interaction and passage of human respiratory epithelial cells may lead to novel compounds and/or approaches in the treatment of lower respiratory tract infections due to *H. influenzae*. Furthermore, the *H*. *influenzae* paracytin may be a valuable tool to study the mechanisms by which epithelial cells maintain intercellular contacts.

Thus, it is envisaged that the nucleotide sequences and/or the proteins identified in the present invention may also find important applications in the field of drug discovery, drug testing and drug development, the development of assays, or in research in general.

Also, the proteins identified in the present invention could be used in the development of vaccines for use in a vaccination strategy for individuals at risk for *H*. *influenzae* infections. Furthermore, antibodies against HI0636 and/or HI0638 could be provided, which may also find therapeutic use and/or use in research applications.

Further aspects and applications of the subject matter of the present invention will become clear from the description given hereinbelow.

The entire genome of *Haemophilus influenzae* strain Rd has been sequenced (vide WO9633276). From computer alignment of the nucleotide sequences of ORFs HI0636 and HI0638 with this entire genomic sequence, it was found that the genomic sequence of *Haemophilus influenzae* strain Rd contains two parts that show a certain degree of homology with the ORFs HI0636 and HI0638 disclosed herein. However, so far, these partial sequences have only been indicated in the art as encoding "hypothetical proteins". Up to the present invention, no biological function and/or activity has been proposed or shown for these partial sequences nor for the proteins encoded by them.

Thus, in a first aspect, the invention relates to isolated nucleic acid encoding a protein or polypeptide that can be used to alter, and in particular increase, the permeability of a tissue or a cell layer, and in particular of an epithelial cell layer such as a lung epithelial cell layer, said nucleic acid having the nucleotide sequence of SEQ ID NO 1 (ORF HI 0636) and/or SEQ ID NO 2 (ORF HI 0638).

The present invention also relates to isolated nucleic acids encoding mutants, variants, analogues, parts and/or fragments of ORF HI0636 or ORF HI0638, as further described below.

In one specific embodiment, these nucleic acids encoding mutants, variants, analogues, parts and/or fragments of ORF HI0636 or ORF HI0638 specifically do not include isolated nucleic acids encoding the sequences of SEQ ID NO 5 or SED ID NO 6, which correspond to the partial sequences found by computer alignment of ORF HI0636 and ORF HI0638 with the entire genomic sequence of *Haemophilus influenzae* strain Rd as disclosed in WO9633276. In this respect, however, it should be noted that ORF HI0636 or ORF HI0638 were obtained from a different strain of *H.influenzae* (A960053 vs Rd) and that the nucleotide sequences of SEQ ID NO 1 and SEQ ID NO 2 encode proteins that differ from the proteins encoded by SEQ ID NO 5 and SEQ ID NO 6.

In particular, the protein encoded by SEQ ID NO 1 differs from the protein of SEQ ID NO 5 in 4 amino acid residues, e.g. in amino acid position 38 a serine residue encoded by SEQ ID NO 5 is replaced by a glycine residue encoded by SEQ ID NO 1, in position 40 a glycine is replaced by a glutamic acid, in position 42 an alanine by a valine and in position 79 an aspartic acid by an asparagine. SEQ ID NO 2 encodes a protein that differs from the protein encoded by SEQ ID NO 6 in 5 amino acid residues, e.g. in amino acid position 33 a glutamic acid residue encoded by SEQ ID NO 6 is replaced by an aspartic acid residue encoded by SEQ ID NO 2, in position 49 an arginine is replaced by a glutamine, in position 50 an isoleucine by a threonine, in position 98 a serine by a proline and in position 139 an isoleucine is replaced by a valine.

Also, it should be noted that WO9633276 only discloses the sequences of SEQ ID NO 5 and SEQ ID NO 6 as part(ial seqeunces) of an entire genomic sequence, and therefore not as isolated nucleic acids.

In a second aspect, the invention relates to an amino acid sequence - e.g. a protein or a polypeptide - encoded by the nucleotide sequences of SEQ ID NO 1/ORF HI0636 and/or SEQ ID NO 2/ORF HI0638. These amino acid sequences - which hereinbelow will also be generally referred to as "paracytin(s)"- are given in SEQ ID NO 3 (HI0636) and SEQ ID NO 4 (HI0638), respectively.

The invention also relates to mutants, variants, analogues, parts and/or fragments of the amino acid sequences HI0636 and/or HI0638, as further described below, and these are also included within the term "paracytin(s)" as used herein.

In one specific embodiment, these mutants, variants, analogues, parts and/or fragments of ORF HI0636 or ORF HI0638 specifically do not include the amino acid sequences encoded by the nucleotide sequences of SEQ ID NO 5 or SED ID NO 6.

In yet another aspect, the invention relates to genetic constructs containing the nucleotide sequences of SEQ ID NO 1 and/or SEQ ID NO 2 or mutants, variants, analogues, part and/or fragments thereof.

Some other non-limiting aspects of the invention relate to some specific uses of the above nucleic acids and/or proteins, to methods for the production of the above proteins, to vaccines containing the above proteins, and to antibodies raised against the above proteins. These and other aspects of the invention be further discussed hereinbelow.

The above nucleic acids/nucleotide sequences were obtained from *Haemophilus. influenzae* strain A960053.

In particular, in the research leading up to the present invention, a chromosomal library of nonencapsulated *H*. *influenzae* strain DNA was constructed in *Escherichia coli* DH5α to identify bacterial genes contributing to this paracytosis. Two *E. coli* clones containing open reading frames (ORFs) homologous to HI0636 up to HI0641 of *H*. *influenzae* strain Rd were identified that showed an increased penetration in epithelial cell layers of the human bronchial epithelial cell line NCI-H292. The two ORF's HI0636 and HI0638 encoding for two small proteins of unknown functions were further investigated. The clone containing ORF HI0636 as well as the clone containing ORF HI0638 showed significant increase in penetration, which demonstrates that these ORFs and/or the proteins encoded by them may influence the permeability of tissues and/or cell layers.

In addition, it was found that disruption of HI0638 by kanamycin box insertion in *H*. *influenzae* strain A960053 resulted in loss of penetration into the epithelial cell layers. Disruption of HI0636 had no effect on penetration in this model system. This may mean that the protein encoded by ORF HI0638 functions as a paracytin, whilst the protein encoded by ORF HI0636 may have an auxiliary function. However, in its broadest sense, the invention is not limited to any specific explanation or mechanism for the way in which HI0636 and/or HI0638 may effect paracytosis.

Besides isolated nucleic acids having the nucleotide sequences given in SEQ ID NO 1 and/or SEQ ID NO 2, the invention also encompasses mutants, variants, analogs, alleles, parts and/or fragments or such nucleic acids/nucleotide sequences.

For instance, such mutants, variants, analogs, alleles, parts and/or fragments may differ from - e.g. may be derived from - the nucleotide sequences of SEQ ID NO.1 and/or SEQ ID NO.2 by addition, substitution, insertion and/or deletion (hereinbelow collectively referred to as *"nucleotide alterations*") of one or more bases/nucleotides at one or more positions.

Preferably, any such mutant, variant, analog, allele, part and/or fragment will have a degree of sequence homology of at least 50%, preferably at least 70%, more preferably at least 90%, and in particular more than 95%, with at least one of the nucleotide sequences of SEQ ID NO.1 and/or SEQ ID NO 2. For this purpose, the percentage of *"sequence homology"* between a given nucleotide sequence and one of the nucleotide sequences of SEQ ID NO 1 and/or SEQ ID NO 2 may generally be calculated by dividing [*the number of bases/nucleotides in the given nucleotide sequence that are identical to the base/nucleotide at the corresponding base position of the nucleotide sequence of SEQ ID NO 1 and/or SEQ ID NO 2, respectively*] by [*the total number of bases/nucleotides in the nucleotide sequence of SEQ ID NO 1 and/or SEQ ID NO 2, respectively* ] and multiplying by [*100%*], in which each deletion, insertion, substitution or addition of a nucleotide in the given sequence - i.e. compared to the sequence of SEQ ID NO 1 and/or SEQ ID NO 2 - is to be considered as a difference at a single base position.

Alternatively, the degree of sequence homology may be calculated using a known computer algorithm for sequence alignment such as BLAST, Align⁺ or PC-gene [*eventueel aanvullen*]. For instance, the degree of sequence homology may be calculated using the BLAST or Align⁺-program at standard settings.

Also, any part or fragment of a nucleic acid/nucleotide sequence of SEQ ID NO.1 and/or SEQ ID NO.2 will preferably contain at least 10%, more preferably at least 30%, even more preferably at least 50%, even more preferably at least 70%, %, even more preferably at least 90%, and in particular at least 95%, of the total number of bases/nucleotides of the full nucleotide sequence of SEQ ID NO 1 and/or SEQ ID NO 2. Also, two or more parts or fragments of a nucleic acid/nucleotide sequence of SEQ ID No.1 (or of SEQ ID No.2) may be joined and/or otherwise combined to provide a nucleotide sequence useful in the invention, in which case the total number of bases/nucleotides present in the combined parts of fragments should again preferably be within the percentages indicated above. Also, any such parts or fragments may contain further nucleotide alterations as defined above, and/or may ligated/fused to other nucleotide sequences, as further described hereinbelow.

Mutants, variants, analogs, parts and/or fragments of the nucleotide sequences of SEQ ID NO.1 and/or SEQ ID NO.2 may also be such that they are capable of hybridizing with at least one of the nucleotide sequences of SEQ ID NO.1 and SEQ ID NO.2 under moderate hybridization conditions, and preferably under stringent hybridization conditions. Such moderate conditions may for instance include: 1 x SSC at 37°C in standard buffers. Such stringent conditions may include: 0,1 x SSC at 65°C in standard buffers.

Also, any such mutants, variants, analogs, parts and/or fragments of the nucleic acid/nucleotide sequences of SEQ ID NO 1 and/or SEQ ID NO 2 are preferably such that the expression thereof in a micro-organism provides said micro-organism with (increased) capacity of paracytosis, as may be determined using a suitable assay, such as an assay involving measurement of the penetration of an epithelial cell layer, for example the assay involving measurement of the penetration of the human bronchial epithelial cell line NCI-H292 described in the Experimental Part.

More preferably, in such an assay, expression of a mutant, variant, analog, allele, part and/or fragment of one of the nucleic acid/nucleotide sequence of SEQ ID NO 1 and/or SEQ ID NO 2 provides the micro-organims with a capacity for paracytosis that is increased by a factor 2, preferably a factor 5, more preferably a factor 10, compared to the same micro-organism in essentially the same assay and under essentially the same conditions but without expression of the nucleic acid/nucleotide sequence of the invention.

Alternatively, in such an assay, the expression of a mutant, variant, analog, allele, part and/or fragment of one of the nucleic acid/nucleotide sequence of SEQ ID NO 1 or SEQ ID NO 2 provides the micro-organisms with a capacity for paracytosis that is at least 50%, preferably at least 80%, and up to 100% or more of the increase in the capacity of paracytosis that is provided to said micro-organism by expression of a nucleic acid/nucleotide sequence of SEQ ID NO 1 and/or SEQ ID NO 2, as measured in essentially the same assay and under essentially the same conditions.

The above isolated nucleic acids may be in the form of DNA and/or of RNA, and may be single stranded or double stranded. Usually, they will be *ds*DNA. Also, the nucleic acids may be in the form of a genetic construct as defined hereinbelow.

The nucleic acids/nucleotide sequences of SEQ ID NO 1 and SEQ ID NO 2 encode proteins/polypeptides that may be used - *in vivo* or *in vitro -* to increase the permeability of tissues or cell layers - and in particular of epithelial cell layers such as lung epitheilial cell layers - and/or to otherwise influence the interaction and/or the intercellular junctions between the cells that form (part of) such tissues or cell layers. The amino acid sequences of these proteins/polypeptides are given in SEQ ID NO 3 (HI0636) and SEQ ID NO 4 (HI0638), respectively.

Compared to other known factors produced by micro-organisms that can effect such an increase of the permeability of tissues or cell layers, such as the toxins produced by *Vibrio cholerae*, *Clostridium difficile* or *Helicobacter pylori* mentioned above, the polypeptides/proteins of the invention (including the mutants, variants, analogs, parts and/or fragments thereof described below) generally have the advantage that they may provide such an increase in permeabilty in a reversible way, essentially without causing a (major) pathologic effect on these tissues or layers.

The invention also relates to mutants, mutants, variants, analogs, parts and/or fragments of the amino acid sequences of SEQ ID NO 3 and/or SEQ ID NO 4.

For instance, such mutants, variants, analogs, parts and/or fragments may differ from the amino acid sequence of SEQ ID NO 3 and/or SEQ ID NO 4 by addition, substitution, insertion and/or deletion (hereinbelow collectively referred to as *"amino acid alterations*") of one or more amino acid residues at one or more positions.

Preferably, such mutants, variants, analogs, parts and/or fragments will have a degree of amino acid homology 50%, preferably at least 70%, more preferably at least 90%, and in particular more than 95%, with the an amino acid sequence of SEQ ID NO 3 and/or SEQ ID NO 4.

For this purpose, the percentage of "amino acid homology" between a given amino acid sequence and one of the amino acid sequences of SEQ ID NO 3 and/or SEQ ID NO 4 may generally be calculated by dividing [*the number of amino acid residues in the given amino acid sequence that are identical to the amino acid residue at the* *corresponding amino acid position of SEQ ID NO 3 and/or SEQ ID NO 4*] by [*the total number of amino acids of SEQ ID NO 3 and/or SEQ ID NO 4, respectively*] and multiplying by [*100%*], in which each deletion, insertion, substitution or addition of an amino acid residue in the given amino acid sequence - i.e. compared to the amino acid sequence of SEQ ID NO 3 and/or SEQ ID NO 4 - is to be considered as a difference at a single amino acid residue.

In this respect, it will be clear to the skilled person that any "conservative" alteration of an amino acid in a sequence will result in an essentially homologues amino acid sequence. Thus for the purpose of this invention amino acid homology also relates to amino acids that are not identical but equal with respect to acidity, basicity, hydrofobicity and hydrophilicity, i.e. substitution of one hydrophobic residue such as isoleucine, valine, leucine or methionine with another hydrophobic residue, or the substitution of one polar residue with another polar residue, such as arginine with lysine, glutamic acid with aspartic acid, or glutamine with asparagine.

Alternatively, the degree of amino acid homology may be calculated using a known computer algorithm for sequence alignment such as BLAST, Align⁺ or PC-gene. For instance, the degree of amino acid homology may be calculated using the BLAST -program at standard settings, optionally taking into account "conservative"amino acid substitutions as mentioned above.

Also, an amino acid sequence encoded by a part of fragment (as defined above) of the amino acid sequence of SEQ ID NO 3 and/or and SEQ ID NO 4 will preferably contain at least 10%, more preferably at least 30%, even more preferably at least 50%, even more preferably at least 70%, even more preferably at least 90 and in particular at least 95% of the total number of amino acid residues encoded by the full nucleotide sequence of SEQ ID NO 3 and/or SEQ ID NO 4. Also, two or more amino acid sequences encoded by two or more parts or fragments of the nucleotide sequences of SEQ ID NO 3 (or SEQ ID NO 4) may be joined and/or otherwise combined to provide a protein or polypeptide useful in the invention, in which case the total number of amino acid residues present in the combined amino acid sequence should again preferably be within the percentages indicated above. Also, any such parts or fragments may contain further amino acid alterations as defined above, and/or may be fused to other amino acid sequences, e.g. as described hereinbelow.

Also, any such mutants, variants, analogs, parts and/or fragments of the amino acid sequences of SEQ ID NO 3 and/or SEQ ID NO 4 are preferably such that they have the capacity to alter, and in particular to increase, the permeability of tissues or cell layers - and in particular of epithelial cell layers such as lung epitheilial cell layers - and/or that may otherwise influence the interaction between cells that form (part of) such tissues or cell layers. This may again be determined in a suitable assay, such as an assay involving measurement of the penetration of an epithelial cell layer, for example the assay involving measurement of the penetration of the human bronchial epithelial cell line NCI-H292 described in the Experimental Part.

More preferably, in such an assay, a mutant, variant, analog, part and/or fragment of the amino acid sequence of SEQ ID NO 3 and/or SEQ ID NO 4 preferably provides an increase in the permeability of a cell layer that is at least 50%, preferably at least 80%, and up to 100% or more of the increase in permeability provided by the amino acid sequence of SEQ ID NO 3 and/or SEQ ID NO 4 in essentially the same assay and under essentially the same conditions.

Generally, such a mutant, variant, analog, part and/or fragment of the amino acid sequence of SEQ ID NO 3 and/or SEQ ID NO 4 will be encoded by a mutant, variant, analog, part and/or fragment of the nucleic acid/nucleotide sequence of SEQ ID NO1 or SEQ ID NO 2. In this respect, however, it will also be clear to the skilled person that, due to the degeneracy of the genetic code, a mutant, variant or analog of a nucleic acid/nucleotide sequence of SEQ ID NO. 1 or SEQ ID No.2 may also encode the same amino acid sequence as given in SEQ ID NO 3 or SEQ ID NO 4, respectively.

In the present invention, the nucleic acids/nucleotide sequences of SEQ ID NO 1 to SEQ ID NO 2 are derived from *Haemophilus influenzae* strain A960053.

However, it is envisaged that on the basis of the disclosure herein, the skilled person will be able to detect and/or isolate nucleic acids/nucleotide sequences similar or corresponding to the nucleotide sequences of SEQ ID NO 1 and/or SEQ ID NO 2 in or from other micro-organisms, in particular from other micro-organisms that show a capacity for paracytosis, more in particular from other species *of Haemophilus,* and even more in particular from other strains of *Haemophilus influenzae.* Such nucleic acids/nucleotide sequences will also be referred to hereinbelow as *"natural homologues"* of SEQ ID NO 1 or SEQ ID NO 2, respectively.

Preferably, such natural homologues of SEQ ID NO 1 and/or SEQ ID NO 2 are as described hereinabove for the mutants, variants, analogues, parts and/or fragments of SEQ ID NO 1 and/or SEQ ID NO 2, in particular with regard to the degree of sequence homology with SEQ ID NO 1 and/or SEQ ID NO 2, with regard to the capability of hybridizing with a nucleotide sequence of SEQ ID NO and/or SEQ ID NO 2, and/or with regard to the biological activity that may be provided by SEQ ID NO 1 and/or SEQ ID NO 2.

Also, such a natural homologue may encode the amino acid sequence of SEQ ID NO 3 and/or SEQ ID NO 4, or may encode a mutant, variant, analog, part and/or fragment of said amino acid sequences (hereinbelow also referred to as *"natural analogs*" of SEQ ID NO 3 or SEQ ID NO 4, respectively). Preferably, such natural analogs of SEQ ID NO 3 and/or SEQ ID NO 4 - which are also included within the term "paracytin(s)"as used herein in its broadest sense - are as described hereinabove for mutants, variants, analogs, parts and/or fragments of SEQ ID NO 3 and/or SEQ ID NO 4, in particular with regard to the degree of amino acid homology with SEQ ID NO 3 and/or SEQ ID NO 4 and/or with regard to the biological activity of SEQ ID NO 3 and/or SEQ ID NO 4.

As already mentioned above, in one embodiment, the natural homologues of SEQ ID NO 1 and/or SEQ ID NO 2 as defined above specifically do not include isolated nucleic acids encoding the sequences of SEQ ID NO 5 and/or SED ID NO 6. Also, another such embodiment, the natural analogs of SEQ ID NO 3 and/or SEQ ID NO 4 as defined above specifically do not include the amino acid sequences encoded by the nucleotide sequences of SEQ ID NO 5 and/or SED ID NO 6.

Generally, based upon the disclosure herein, such natural homologues and/or natural analogues may be identifed from known (genomic) sequences of micro-organisms, including but not limited to the sequences compiled in known databases. This may for instance be carried out using known computer algorithms, set according to the parameters for sequence/amino acid homology set out hereinabove. It will also be clear that the sequence of SEQ ID NO 1, of SEQ ID NO 2 and/or any part(s) thereof may also be used as a probe for detecting/isolating such natural homologues from a suitable biological source.

For instance, with respect to SEQ ID NO.2, which is *Haemophilus influenzae* gene HI0638 from *H. influenzae* strain A960053, encoding a protein of 205 amino acids, some possible natural homologues have been identified from the database "NCBI microbial genomes, finished and unfinished", using a standard BLAST search. These are given in Table 1.

**Table 1**

| | amino acids | percent Identity/positives: |
|---|---|---|
| *Actinobacillus actinomycetemcomitans*: | 1-204 | 57/67 |
| *Yersinia pestis* | 2-200 | 53/69 |
| *Escherichia coli k-12* | 2-204 | 51/66 |
| *Salmonella typhi* | 2-200 | 51/66 |
| *Salmonella typhimurium LT2* | 60-204 | 51/68 |
| | 2-56 | 49/65 |
| *Klebsiella pneumoniae* | 129-204 | 52/71 |
| | 2-78 | 50/64 |
| | 72-127 | 51/63 |
| *Vibrio cholerae* | 5-204 | 41/64 |
| *Salmonella typhimurium LT2* | 2-154 | 50/65 |
| *Shewanella putrefaciens* | 5-199 | 38/64 |
| *Pseudomonas aruginosa* | 8-194 | 35/56 |
| *Pasteurella multocida PM70* | 131-204 | 67/84 |
| *Thiobacillus ferrooxidans* | 6-203 | 28/49 |
| *Salmonella paratyphi A* | 158-188 | 61/80 |
| | 2-203 | 26/41 |
| | 16-116 | 22/45 |
| *Neisseria meningitidis* | 42-92 | 35/52 |
| *Neisseria gonrrhoeae* | 42-92 | 35/52 |

In Table 1, the percentage positive amino acids corresponds to the amino acids that are identical and the amino acids that are not identical but equal with respect to acidity, basicity, hydrofobicity and hydrophilicity (e.g. "conservative" substitutions as mentioned above.)

The nucleic acids of SEQ ID NO 1 and/or SEQ ID NO 2 and any natural homologues thereof may be isolated from their respective natural source(s), e.g *H*. *influenzae* strain A960053 for the nucleic acid of SEQ ID NO 1 and/or SEQ ID NO 2.

Mutants, variants, analogs, parts and/or fragments of these nucleic acids may for instance be provided by recombinant techniques known per se, including but not limited to automated DNA synthesis; site-directed mutagenesis; a PCR reaction with one or more "mismatched" primers using - for instance - the nucleic acids of SEQ ID NO.1 and/or SEQ ID NO 2 as a template; combining two or more parts of one or more of these nucleotide sequences (e.g. using one or more restriction enzymes and/or ligases); introduction of mutations that lead to the expression of a truncated expression product; or any suitable combination of such techniques. These and other suitable techniques are for instance described in Sambrook et al, "Molecular Cloning: A Laboratory Manual" ( 2nd.ed.), Vols. 1-3, Cold Spring Harbor Laboratory (1989) of F. Ausubel et al, eds., "Current protocols in molecular biology", Green Publishing and Wiley Interscience, New York (1987) and Saiki et al., Science 239 (1988), 487-491 or PCR Protocols, 1990, Academic Press, San Diego, California, USA.

In a further aspect, the invention relates to a genetic construct, said construct at least comprising a nucleotide sequence of SEQ ID NO.1 and/or SEQ ID NO.2 - or a mutant, variant, analogue, part, fragment and/or natural homologue thereof - and optionally one or more elements of genetic constructs known per se.

Such a genetic construct may be in the form of a DNA sequence or an RNA sequence, and is preferably in the form of a double stranded DNA sequence. It may also be in the form of plasmid or vector; and/or in a form suitable for transforming host cell, e.g. a micro-organism.

The genetic construct may further be such that, upon transformation into a micro-organism, it is stably maintained, replicated and/or inherited in said host micro-organism. Alternatively, the genetic construct may also be such that - upon transformation - the entire construct or at least part thereof is integrated into the DNA of the host micro-organism

The *"optional further elements"* that may be present in the genetic constructs of the invention include, but are not limited to, one or more expression regulating sequences such as a promoter, leader sequences, terminators, enhancers, integration factors, selection markers and/or reporter genes. Preferably, any such optional further elements are *"operably linked"* to the nucleotide sequence of the invention and/or to each other, by which is generally meant that they are in a functional relationship with each other. For instance, a promoter is operably linked to a coding sequence (e.g. the sequence of SEQ ID NO 1 and/or SEQ ID NO 2) if the promoter is able to initiate or otherwise control/regulate the transcription and/or expression of a coding sequence (in which said coding sequence should be understood as being *"under the control of*" said promotor)

Generally, when two nucleotide sequences are operably linked, they will be in the same orientation and usually also in the same reading frame. They will usually also be essentially contiguous, although this may not be required. Preferably, the optional further elements of the genetic construct are further such that they are capable of providing their intended biological function in the host cell or host organism.

The construct of the invention can be provided in a manner known per se, which generally involves techniques such as restricting and linking nucleic acids/nucleic acid sequences, for which again reference is made to the standard handbooks, such as Sambrook et al. and/or F. Ausubel et al., both mentioned above.

The genetic constructs of the invention may be used to transform a suitable host (cell) and in particular a micro-organism, for instance to provide said micro-organims with (increased) capacity for paracytosis and/or to provide micro-organisms suitable for the (heterologous) production of the proteins/polypeptides mentioned above.

Thus, in a further aspect, the invention relates to a method for providing a host cell, and in particular a cell of a micro-organism, with (increased) capacity for paracytosis, said method at least comprising transforming said host cell with at least one nucleic acid as described hereinabove, and in particular with at least one genetic construct as mentioned hereinabove. In another aspect, the invention relates to the transformed micro-organism thus obtained.

In yet another aspect, the invention relates to a method for the production of HI0636 and/or HI0638 - or a mutant, variant, analogue, part, fragment and/or natural analog thereof - said method at least comprising:
- transforming a host cell with at least one nucleic acid encoding HI0636, HI0638 or a mutant, variant, analogue, part, fragment and/or natural analog thereof; and in particular with a genetic construct as mentioned hereinabove;
- maintaining the host cell thus transformed under conditions such that said nucleic acid is expressed by said host cell; and optionally
- recovering the protein/polypeptide thus expressed, e.g. from the host cell and/or from the medium.

Suitable techniques, genetic constructs and host organisms for such (heterologous) production will be clear to the skilled person, e.g. from Sambrook et al. and/or from Ausubel et al., both mentioned above.

Yet other aspects of the invention therefore relate to transformed host cells/micro-organisms suitable for the (heterologous) production of the above proteins/polypeptides, as well as to the proteins/polypeptides thus obtained.

Generally, the invention comprises both *in vitro* as well as *in vivo* applications of the nucleic acids/nucleotide sequences and/or the amino acid sequences mentioned above, e.g. (the sequence encoding) HI0638 and/or HI0638, any natural homologues or analogues thereof (as defined above); and/or any mutants, analogs, variants, parts and/or fragments thereof (also as defined above).

For instance, one such possible *in vivo* and/or *in vitro* application comprises the use of one or both proteins HI0636 and/or HI0638 (or more generally the parayctins disclosed herein) in altering, and in particular in increasing, the permeability of tissues and/or cell layers, and in particular of epithelial cell layers such as lung epithelial layers.

For example, the paracytins of the invention may be used in (the formulation of) pharmaceutical preparations, in particular in (the formulation of) pharmaceutical preparations for the administration of one or more active substances or principles across a cell layer, such as an epithelial cell layer or an endothelial cell layer, for instance the lung epithelial cell layer or the epithelial cell layer of the gastro-intestinal tract, or the endothelial cell layer of blood vessels or the umbilical cord.

Thus, in another aspect, the invention relates to a pharmaceutical composition, comprising at least one therapeutically active substance and one or both of the proteins HI0636 or HI0638; or mutants, variants, analogues, parts and/or fragments thereof (as defined herein).

In particular, such a pharmaceutical composition will contain the one or both of the proteins HI0636 or HI0638 (and/or one or more mutants, variants, analogues, parts and/or fragments thereof ) in an amount that is sufficient to alter, and in particular increase, the permeability of an epithelial cell layer for said at least one therapeutically active substance.

The above pharmaceutical compositions may be formulated in any manner known per se, depending upon the intended route of administration. Usually, this route of administration will be such that the active principle has to cross at least one cell layer, and in particular an epithelial cell layer such as the lung epithelial layer. Thus, the pharmaceutical composition may be specifically formulated for such a route of administration, optionally using one or more pharmaceutically acceptable carriers, adjuvantia and/or exipientia.

It is also envisaged that paracytins of the invention may find use as additives in pharmaceutical preparations for the administration of therapeutically active substances to the lungs and/or the respiratory tract, e.g. for the treatment of disorders of the lungs and/or the respiratory tract. In such preparations, the paracytins of the invention may for instance increase and/or prolong the effectiveness of the therapeutically active substance(s) present or otherwise beneficially influence the therapeutic and/or pharmacological properties thereof. Again, such preparations may be formulated in a manner known per se.

In the above pharmaceutical applications of the paracytins of the invention, it is also envisaged that in a first step, said paracytins are administered to a subject - for instance to increase the permeability of an epithelial cell layer of said subject - and that (some time) thereafter, e.g. in a second step, (the composition containing) the therapeutically active substance is administered, i.e. essentially via the same route of administration.

Thus, another aspect of the invention relates to a pharmaceutical composition for altering, and in particular increasing, in a subject the permeability of at least one epithelial cell layer, said composition comprising one or both of the proteins HI0636 or HI0638 ( or one or more mutants, variants, analogues, parts and/or fragments thereof), in particular in an amount that is sufficient to alter, and in particular increase, the permeability of an epithelial cell layer in said subject.

Again, such a composition may be formulated in any manner known per se, depending upon the intended route of administration, and optionally using one or more pharmaceutically acceptable carriers, adjuvantia and/or exipientia. Such a composition may also be provided as a kit of parts with a pharmaceutical composition containing the active substance(s) to be administered.

In another aspect, the invention encompasses the use of nucleotide sequences encoding the paracytins of the invention in (the preparation of) gene therapy vectors. In such gene therapy vectors, the nucleotide sequences encoding the paracytins may in particular be used to alter, and more in particular increase, the permeability of a tissue to which said gene therapy agent is administered, e.g. for a therapeutically active substance also encoded by said gene therapy vector.

More generally, it is also envisaged that (the nucleotide sequences encoding) the paracytins of the invention may otherwise be used to make tissues more permeable for for instance therapeutic agents and/or therapeutic intervention. For instance, the proteins or nucleotide sequences - e.g. in the form of a gene therapy vector - could be used to make (solid) tumors more accessible for treatment, e.g. with cytostatic agents, and/or to improve or otherwise facilitate the transport of active principles across the walls/lining of blood vessels.

Also, it is envisaged that the nucleotide sequences encoding paracytins of the invention could be used in providing - e.g. via suitable recombinant techniques-fusions of said paracytins with one or more further amino acid sequences, such as therapeutically active proteins or polypeptides. Again, this may favorably influence the therapeutic and/or pharmacological properties of such an amino acid sequence, e.g. in essentially the same manner as described hereinabove.

It is also envisaged that (the nucleotide sequences encoding) the paracytins of the invention may be used in (the development of) *in vivo* and/or *in vitro* assays; for the study of biological processes and/or mechanisms, for instance in order to obtain insight into the invasion mechanisms used by various bacteria; in drug discovery, drug development and/or drug testing; and/or for research purposes in general.

In yet another application, the paracytins of the invention may be used in the preparation of a vaccine against *H. influenzae.* Such vaccines, which form a further aspect of the invention, will generally contain an amount of a paracytin of the invention sufficient to ilicit a protective immune response in a subject to which said vaccine is administered (e.g. a human or a mammal). Such vaccines may be administered in a manner known per se, including but not limited to administration into the lungs, and may also be formulated in a manner known per se suitable for such administration, again optionally using one or more pharmaceutically acceptable carriers, adjuvantia and/or exipientia.

Yet another application of the invention that is envisaged comprises the use of paracytosis "knock-out" mutants, e.g. microorganisms including but not limited to strains of *H. influenzae* in/from which the native paracytose activity provided by for instance HI0636 and/or HI0638 has been reduced or even essentially fully inhibited/removed, for example by disruption of the ORFs/genes encoding HI0636 and/or HI0638.

More generally, such "knock-out" mutants may be provided by, in a given micro-organism, reducing and preferably fully removing the paracytosis activity (natively) provided by any natural homologue (as defined above) of the ORFs HI0636 and/or HI0638 that may be (natively) present in said microorganism.

Such parayctosis "knock-out" mutants may for instance be used in the preparation of live attenuated vaccines. Thus, another aspect of the invention relates to a vaccine, in particular a live attenuated vaccine, based upon and/or containing such a paracytosis "knock-out" mutant, including but not limited to a HI0636 and/or HI0638 "knock out" *H. influenzae* mutant.

The paracytins of the invention may also be used to obtain (monoclonal) antibodies directed against these paracytins, and such antibodies form a further aspect of the invention. Such antibodies may be generated in a manner known per se, such as immunisation of a rabbit, mouse or other mammal and followed by harvesting the antibodies thus ilicited. These antibodies may again be used therapeutically and/or for research purposes, as will be clear to the skilled person.

The nucleotide sequences encoding the paracytins of the invention may also be used to provide micro-organimsm with (enhanced) capability of paracytosis. This will generally involve transforming said micro-organism with at least one nucleotide sequence encoding a paracytin of the invention, e.g. using a genetic construct as described hereinabove.

In ths aspect, the invention is especially relevant in combination with one or more other characteristics that are incorporated in the micro-organism. Such other characteristics could for instance be agents, such as dyes or histological agents which allow study of how cells are connected and of permeability inbetween cells. Also therapeutic agents, such as radioisotopes, therapeutic surface proteins, for instance (monoclonal) antibodies could be incorporated in the micro-organism. According to the method of the invention the micro-organism is capable to penetrate in an epithelial cell layer, thereby transporting the therapeutic agent to a position it would normally not reach.

As mentioned above, the invention is not limited to any specific explanation and/or mechanism as to the way in which the paracytins of the invention may effect paracytosis. Also, to cause said paracytosis and/or for any of the applications mentioned above, only the presence of HI0636, only the presence of HI0638, or the presence of both may be required/desirable.

However, as also indicated above, it may be that the protein encoded by ORF HI0638 functions as a paracytin, whilst the protein encoded by ORF HI0636 may have an auxiliary function in the paracytosis effected by HI0638. Thus, according to one aspect, the invention involves the use of either HI0638 by itself or the use of HI0638 in combintion of HI0636. However, it should be noted that any such activity of HI0636 as an auxiliary factor involved in paracytosis may also make this factor as such (i.e. by itself without HI0638) suitable for use in one or more of the applications mentioned above.

The invention will now be illustrated by means of the following non-limiting Experimental Part, as well as the Figures, in which:
- Figure 1 is a schematic representation showing the relative positions of the primers on the chromosomal fragment of clone 9.1;
- Figure 2 is a graph/diagram showing the number of bacteria cultured from of the lysed epithelial cell layers after paracytosis, 4 h postinoculation with *H*. *influenzae* strains A960053 (CF), A960005 (COPD) and Rd and *E. coli* DH5α containing plasmid pGJB103, expressed as the number of bacteria in CFU/ml. P values as determined with Students t-test.
- Figure 3 is a graph/diagram showing the number of bacteria cultured from of the lysed epithelial cell layers after paracytosis, 4 h postinoculation with *E. coli* DH5α expressing pGJB103, and *E. coli* clones 9.8 and 9.1. P values as determined with the paired t-test.
- Figures 4A and 4B are light micrographs of cross sections of NCI-H292 cell layers on filter inserts after incubation with *E*. *coli* clones obtained after selection for paracytosis. (A) Cell layer 4 h postinoculation with an hmw-positive adherent clone from the chromosomal library of *H*. *influenzae* strain A950006. (B) Cell layer 4 h postinoculation with *E. coli* clone 9.1, obtained from the chromosomal library of *H*. *influenzae* strain A960053.
- Figure 5 is a schematic representation of clones 9.1 and 9.8 and the subclones obtained from clone 9.1.
- Figure 6 is a graph/diagram showing the number of bacteria cultured from of the lysed epithelial cell layers after paracytosis, 4 h postinoculation with *E. coli* DH5α expressing pUC19, and *E*. *coli* clones 9.1A, 9.1B and 9.1H. P-values as determined with paired t-test.
- Figure 7 is a graph/daigram showing the number of bacteria cultured from of the apical medium (●) or from the lysed epithelial cell layers after paracytosis (○), 4 h postinoculation with *H. influenzae* A960053 and its HI0636 and HI0638 knock out mutant, and *H. influenzae* strain CF47A. P-values as determined with the Students t-test.

### Experimental Part:

*Haemophilus influenzae* penetrates between epithelial cells via an unknown mechanism. A chromosomal library of nonencapsulated *H. influenzae* strain A960053 DNA was constructed in *Escherichia coli* DH5α to identify bacterial genes contributing to this paracytosis. Two *E. coli* clones containing open reading frames (ORFs) homologous to HI0636 up to HI0641 of *H. influenzae* strain Rd were identified that showed an increased penetration in epithelial cell layers of the human bronchial epithelial cell line NCI-H292. The two ORF's HI0636 and HI0638 encoding for two small proteins of unknown functions were further investigated. The clone containing ORF HI0636 as well as the clone containing ORF HI0638 showed significant increase in penetration. Disruption of HI0638 by kanamycin box insertion in *H*. *influenzae* strain A960053 resulted in loss of penetration into the epithelial cell layers. Disruption of HI0636 had no effect on penetration in this model system. This may indicate that the protein encoded by ORF HI0638 may function as a paracytin, whilst HI0636 may have an auxiliary function.

### A. Introduction

*Haemophilus influenzae* is a natural inhabitant of the human upper respiratory tract. Encapsulated strains, in particular those with a serotype b polysaccharide, are important pathogens causing systemic disease such as meningitis, epiglottitis, cellulitis, arthritis, sepsis and pneumonia. Nonencapsulated (nontypeable) *H*. *influenzae* is a frequent cause of respiratory tract infections including otitis media, sinusitis, and persists in the lower respiratory tracts of patients with chronic obstructive pulmonary disease (COPD) and cystic fibrosis (CF). Vaccination of large groups of children with a *H. influenzae* type b conjugate vaccine eradicated systemic disease, and also reduced oropharyngeal carriage of *H*. *influenzae* type b. However, antibodies against *H. influenzae* type b are not effective against nontypeable *H. influenzae* since these bacteria lack the capsule antigen.

Passage of *H. influenzae* type b as well as nontypeable *H. influenzae* through the nasopharyngeal epithelium is assumed to occur during carriage as well as prior to systemic disease. Also during chronic lower respiratory tract infections in CF and COPD patients *H*. *influenzae* was detected between the epithelial cells and in the subepithelial layers in lung tissue. Infections in these patients are common despite the presence of specific antibodies, suggesting that *H*. *influenzae* can circumvent antibody-mediated defense mechanisms. Findings obtained with tissue culture model studies support the *in vivo* observations that *H. influenzae* penetrates between epithelial cells and resides beneath and between these cells. The effect of bactericidal antibiotics, and bactericidal activity mediated by specific antibodies in the presence of complement on *H. influenzae* concealed in epithelial cell layers was significant less compared to *H*. *influenzae* in the apical fluid. Therefore, penetration between the epithelial cells may play an important role in the persistence of *H. influenzae* in the lower respiratory tract.

Herein, a library of chromosomal DNA of nontypeable *H. influenzae* strain A960053 in *E. coli* to identify genes of *H. influenzae* involved in paracytosis was constructed. Clones penetrating between epithelial cells were subcloned and two genes likely involved in paracytosis were disrupted in nontypeable *H. influenzae* strain A960053. It is also shown that disruption of ORF HI0638 in this strain reduced paracytosis through the epithelial cells.

### B. Materials and methods

**Bacterial strains and plasmids.** Nonencapsulated *H. influenzae* strains A960053 and CF47A were isolated from sputum samples of CF patients, and *H. influenzae* strain A950006 was obtained from the sputum of a COPD patient. For cloning purposes, *E.coli* strain DH5a and *E.coli* strain NM522 and the plasmids pGJB103 (Tomb, J.F. et al. 1989 J.Bacteriol. 3796-8022), pUC21 (Vieira, J. and J. Messing. 1991 Gene 100:189-194), pUC19 and pBR322 were used. The TA-vector pCR2.1, was supplied with the TA cloning kit (In vitrogen, Leek, The Netherlands). pUC19ΔHS was constructed by digesting pUC19 with HindIII and SmaI followed by self ligation.

The MIC for gentamicin of the strains used was determined by E-test (AB Biodisk, Sweden). Adherence of *H. influenzae* to the NCI-H292 epithelial cells was determined as described previously (van Schilfgaarde, M., L. et al. 1995 Infect.Immun. 63:4729-4737). Strain A960053 was nonadherent since less than 5 bacteria per cell were observed. Strain A960005 adhered to the cell line since 50-200 bacteria bound per cell.

**Growth conditions.** *H. influenzae* strains were cultured overnight on chocolate agar plates at 37°C in humid air enriched with 5% CO₂. *E*. *coli* strains were grown on LB agar plates. Strains were stored at -70°C in peptone containing 15% glycerol. Standard concentrations of antibiotics were 100 µg/ml for ampicillin (pUC vectors), 12.5 µg/ml for tetracyclin (pGJB103), and 50 µg/ml for kanamycin (pBR322-kana). The kanamycin resistant knockout mutants of *H*. *influenzae* A960053 were grown in the presence of 20 µg/ml kanamycin.

**Cell culture.** The epithelial cell line NCI-H292 (ATCC CRL 1848) (Banks-Schlegel, S.P. et al. 1985 Cancer Res. 45:1187-1197) originating from a human lung mucoepidermoid carcinoma, was maintained in HEPES buffered RPMI 1640 medium (Gibco, Life Technologies, Breda, The Netherlands), supplemented with 10% fetal calf serum (Boehringer, Mannheim, Germany) without antibiotics (RPMI) and passaged as described previously (van Schilfgaarde, M., L. et al. 1995 Infect.Immun. 63:4729-4737). Cell layers for paracytosis assays were prepared on 10 mm tissue culture insert (Nunc, Roskilde, Denmark) with 0.2 µm pores as described (van Schilfgaarde, M., P. et al. 1999 Microb Pathog 26:249-262).

**Paracytosis assay.** The number of bacteria present between the epithelial cells was determined as the number of bacteria surviving gentamicin treatment of the cell layer after infection of the cell layer as described previously (van Schilfgaarde, M., P. et al. 1999 Microb Pathog 26:249-262). Briefly, *H*. *influenzae* or *E. coli* grown overnight on chocolate or LB agar plates respectively were suspended in PBS to an OD₆₀₀= 1 and 40 µl of the bacterial suspension was added to 360 µl apical fluid. The infected cell layers were incubated at 37°C in a CO₂-incubator for 4 h or overnight as indicated. Then, the infected cell layers were washed three times with 200 µl RPMI and incubated with 450 µl RPMI containing gentamicin (Centrafarm). *H. influenzae* strains were exposed to 200 µg/ml for 2 h, and *E. coli* DH5α to 50 µg/ml for 1 h. The concentrations used were at least 10 times the MIC's of the strains. The apical fluid was removed and the number of bacteria was determined. The cell layers were again washed and lysed with 1% saponin solution in PBS. The number of bacteria in the various fractions was determined by counting the number of CFU/ml on chocolate agar plates of appropriate dilutions of samples of the fractions. Alternatively, cross sections of the cell layers were examined by light microscopy or TEM. These sections were prepared as described before (van Schilfgaarde, M., L. et al. 1995 Infect.Immun. 63:4729-4737).

**Construction of a chromosomal library of *H. influenzae* in *E*. *coli* DH5α.** *H*. *influenzae* chromosomal DNA was isolated by the phenol-chloroform method (Langenberg, W. et al. 1986 J.Clin.Microbiol. 414-477). Sau3A partial restriction digests of chromosomal DNA were separated by agarose gel electrophoresis, and DNA fragments in the 8 to 12- kb range were used to construct a library by ligation into the unique BglII-site of pGJB103 (Tomb, J.F. et al. 1989 J.Bacteriol. 3796-8022). The ligation mixture was transformed to *E. coli* DH5α by electroporation.

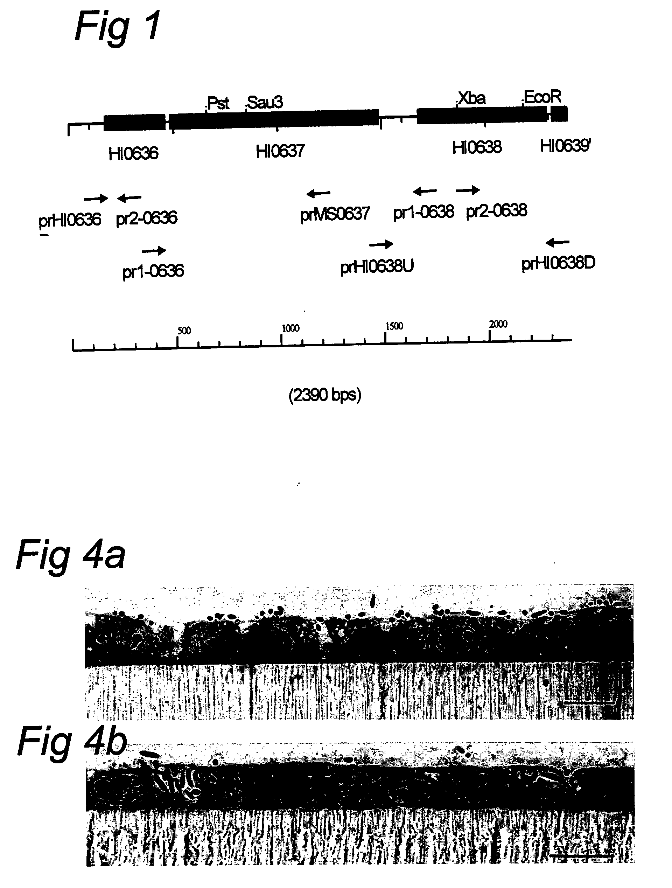

**Subcloning techniques.** Plasmids were isolated using the Wizard Plus SV minipreps kit (Promega). Restriction endonuclease digestions and gel electrophoresis were performed according to standard techniques (Sambrook, J. et al., 1989. Molecuar Cloning: A Laoratory Manual, AnonymousCold Spring Harbor Laboratory Press, Cold Spring Harbor, NY). Fragments were isolated from the agarose gels using the QIAquick gel extraction kit (Qiagen). DNA ligations were done according to standard techniques (Sambrook), with addition of 1 mM ATP. Ligated plasmids were introduced into *E.coli* NM522 by electroporation, and later transformed to *E. coli* DH5α. Transformants containing pUC 19 plus an insert were selected by the blue/white screening on LB plates containing IPTG/Xgal and 100 µg/ml ampicillin. The plasmids were isolated and transformed to *E. coli* DH5α. PCR reactions were performed using the *Ready To Go* beads (Pharmacia Biotech). **Construction of HI0636 and HI0638 knock out mutants of strain A960053.** HI0636 and HI0638 knockout mutants of *H*. *influenzae* strain A960053 were constructed by insertion of the kanamycin resistance gene from pBR322-kana into the genes by homologous recombination. To clone the ORF's HI0636 and HI0638 with the flanking regions, several oligonucleotide primers were generated as indicated in Table 2 and Fig.1. To construct the kanamycin resistance gene into ORF HI0636, the 2 kb product obtained by PCR with primer pr2-0636BAM on chromosomal DNA of *H. influenzae* strain A960053 was cloned into the TA vector and transformed to *E. coli,* resulting in clone pHIP12. Using primers pr1-0636BAM and pr1-0638 in a PCR on clone 9.1 the sequence downstream of HI0636 was amplified. The resulting product was purified from gel and ligated into the TA vector which was transformed to *E. coli* resulting in clone pHIP13. The cloned fragment was obtained from pHIP13 by digestion with XbaI and BamHI and ligated into XbaI and BamHI digested pUC21 together with the kanamycin box from BamHI digested pBR322-kana resulting in clone pHIP15b. The upstream region of HI0636 was ligated into the BamHI site upstream of the kanamycin box in clone pHIP15b, resulting in clone pHIP15. To construct the kanamycin resistance gene into ORF HI0638, a 3 kb PstI fragment from clone 9.1 containing HI0638 and approximately 1 kb flanking region at both sides was ligated in pUC19 and transformed to *E. coli*, resulting in clone 0638FL. To obtain a BamHI restriction site in HI0638, the upstream and downstream sequences of HI0638 were amplified separately by performing PCR reactions with the primer sets M13forward/pr1-0638BAM and M13reversed/pr2-0638BAM on clone 0638F1. Both PCR products were purified from gel and cloned into the TA vector resulting in pHIP10 and pHIP11 respectively. The cloned fragment of pHIP11 was obtained by digestion with PstI and BamHI and ligated into PstI and BamHI digested pUC19 resulting in clone pHIP14a. The cloned fragment of pHIP10 was obtained by digestion with SacI and BamHI and ligated into SacI and BamHI digested pHIP14a resulting in pHIP14b. The kanamycin box was obtained from pBR322-kana by digestion with BamHI and ligated into the BamHI site in pHIP14b resulting in pHIP14. Plasmids pHIP15 and pHIP14 were linearized with NdeI, and transformed to *H. influenzae* strain A960053 using the method described by Herriott et al. (Herriott, R.M. et al., 1970 J.Bacteriol. 101:517-524) resulting in kanamycin resistant colonies. The presence of the kanamycin box in HI0636 or HI0638 was checked by PCR with the primer combination prHI0636D/prMS0637 and prHI0638UP/prHI0638D, respectively.

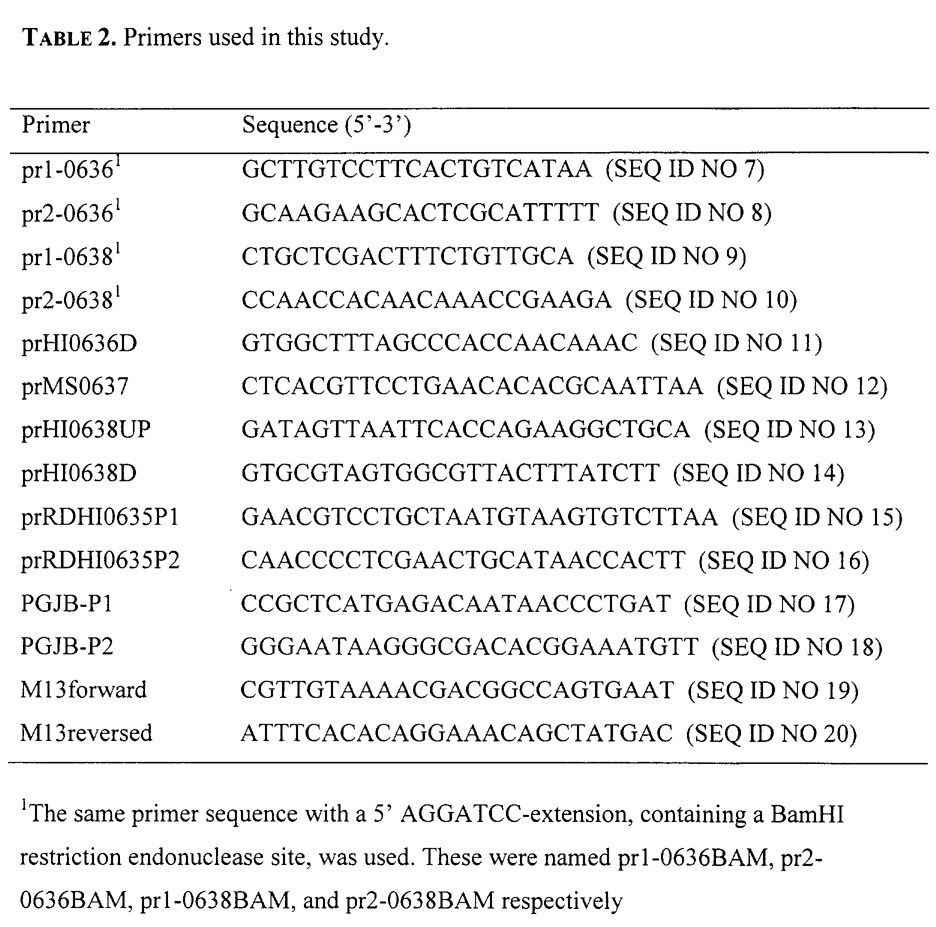

**DNA sequence analysis.** DNA sequence analysis was performed using the big dye terminator cycle sequencing ready reaction kit (Perkin Elmer, Great Britain) according to the manufacturer description. The primers pGJB-P1 and pGJB-P2 hybridizing to regions near the Bgl II site of pGJB103 were designed to sequence the chromosomal fragment in the pGJB103 clone (Table 2). The sequences of several subclones in pUC19 were determined using the M13forward and M13reversed primers. DNA sequence analysis was performed with an automated fluorescent DNA sequencer, model 310 (Perkin Elmer). Data were analyzed with Auto assembler 2.0, ABI Prism, and aligned with known sequences using Clone Manager 5.0. The sequences of *H*. *influenzae* strain A960053 were in most cases compared to sequences of *H. influenzae* Rd strain Kw20, of which the complete genome was sequenced (Fleischmann, R.D. et al., 1995 Science 269:496-512) which is available in GenBank (accession L42023).

**Statistics.** Data on the number of bacteria in the paracytosis assays were evaluated using the student t-test. The data on the *E*. *coli* clones were evaluated pairwise because of a large day to day difference in the number of bacteria of the negative control isolated from the cell layer. With the paired t-test, each pair consisted of a testclone and the negative control in the same experiment. P-values of <0.05 were considered statistically significant.

### C. Results:

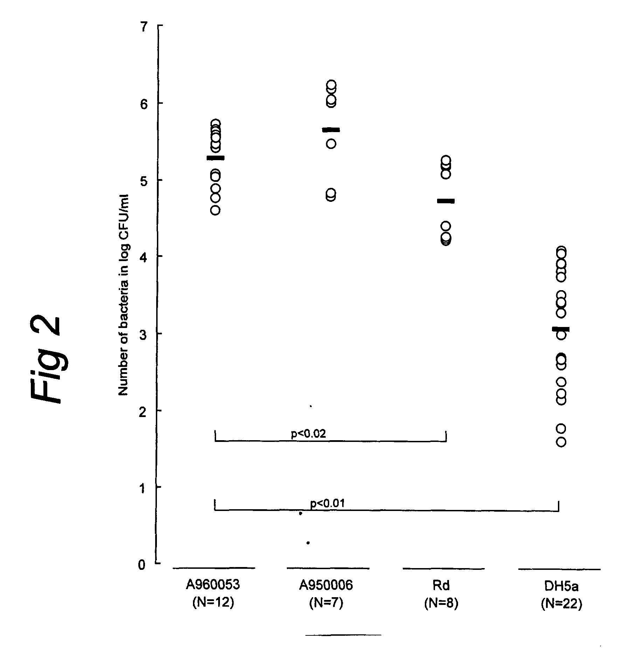

**Survival of bacteria in the cell layer as measured by gentamicin assay.** To identify the bacterial component responsible for paracytosis we chose to clone a library of *H*. *influenzae* chromosomal DNA in a paracytosis negative bacterium. Two clinical *H. influenzae* isolates, strains A960053 and A950006 were used as donor strains, since *H. influenzae* strain A960053 did not adhere to the cell line, and *H. influenzae* strain A950006 was well adherent. *H. influenzae* strain Rdrec1 containing pGJB103 and *E. coli* strain DH5α containing pGJB103 were tested to see if they could be used as recipient for cloning. Selection of clones penetrating into the epithelial cell layers, determined by survival in the presence of gentamicin, was described before (van Schilfgaarde, M., P. et al. 1999 Microb Pathog 26:249-262). In order to test the chromosomal library in *E*. *coli* DH5α we adjusted the incubation time from 24 h to 4 h to prevent damage of the cell layer by *E*. *coli.* In Fig. 2 the number of bacteria in the cell layer after infection for 4 h of the three *H. influenzae* strains and *E*. *coli* containing pGJB103 is depicted. Approximately 10⁵ CFU/ml of the cell-associated bacteria of the three *H*. *influenzae* strains A960053, A950006 and Rd were found, whereas no bacteria in the apical medium survived the gentamicin treatment, indicating that these *H. influenzae* strains had penetrated into the cell layer. The number of bacteria of *H*. *influenzae* strain A950006 was slightly higher than the number of bacteria of strain A960053, which is probably due to adherence of strain A960005 to the cells. Although the number of bacteria of strain Rd from the cell layer was significantly lower than that of strains A960053 and A950006, we thought that the difference in numbers of bacteria between Rd and the clinical isolates was too small to use Rd as a recipient for the chromosomal library. The number of bacteria recovered from the cell layer after infection with *E. coli* DH5α was 10-100 fold lower compared to that of the *H. influenzae* strains, and *E. coli* DH5α was therefore used for further cloning.

**Selection of** *E.coli* **DH5α clones with elevated level of paracytosis.** Since the adherent *H. influenzae* strain A950006 showed slightly higher numbers of bacteria penetrating in the cell layers and adherent strains were shown earlier to pass the cell layers in larger amounts (van Schilfgaarde, M., P. et al. 1999 Microb Pathog 26:249-262; van Schilfgaarde, M., L. et al. 1995 Infect.Immun. 63:4729-4737) we cloned a chromosomal library of A950006 DNA in *E. coli* DH5α. The adhesin of A950006 was identified earlier as a HMW protein. Selection of this library by three subsequent paracytosis enrichment cycles, led to identification of 2 clones containing plasmids with different restriction endonuclease patterns showing a 10 fold higher penetration rate when tested seperately (data not shown). However, these 2 clones and 5 clones from another selection round were positive for *hmwA* when tested in a PCR, indicating that this adhesin facilitates penetration of *E*. *coli.*

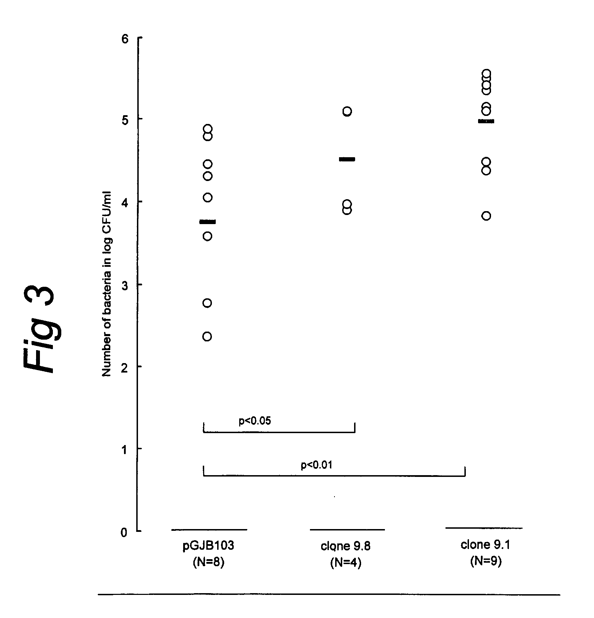

To identify the *H*. *influenzae* genes involved in penetration irrespective of adherence we cloned a chromosomal library of the nonadherent *H*. *influenzae* strain A960053 into *E*. *coli.* A library consisting of approximately 15000 clones was obtained. The library was screened for clones that entered the epithelial cell layer by three subsequent paracytosis enrichment cycles on NCI-H292 cells. After the selection the plasmids of 15 clones were analyzed by restriction enzyme analysis. Of the 15 clones 3 contained an empty vector. Eight of the 15 clones contained the same fragment, referred to as 9.1, and two others contained fragment 9.8. Two clones contained different fragments. When tested separately, both clone 9.1 and clone 9.8 survived in the cell layers in higher numbers than *E. coli* DH5a containing only pGJB103 (p<0.01 and p<0.05, respectively) (Fig. 3). Since the MIC for gentamicin of the clones was unaltered, this result indicates that the higher survival of these two clones in the cell system in the presence of gentamycin was the consequence of paracytosis of the clones.

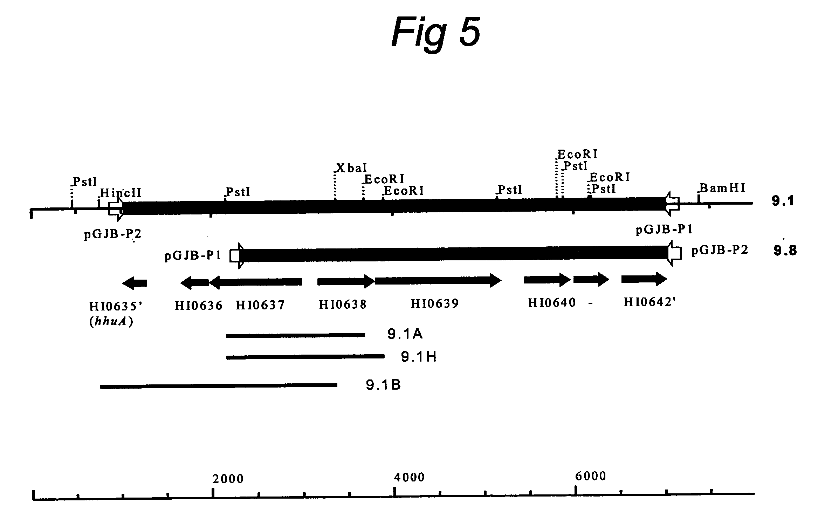

Sections of the cell layers on the permeable support incubated with the *E.coli* clones for 4 h were screened by light microscopy for the presence of bacteria. An *hmw*-positive adherent clone, selected from the chromosomal library of strain A950006 was found occasionally between the cells , but predominantly on the surface of the cell layer (Fig. 4a). In contrast, *E. coli* clone 9.1 was found predominantly between the epithelial cells and few bacteria were detected on top of the cell layer (Fig. 4b). We observed no bacteria in sections of epithelial cell layers exposed to *E. coli* containing the empty plasmid pGJB103 for 4 h (data not shown). These data show that the *E. coli* clone 9.1 penetrated between the cells irrespective of the bacterial ability to adhere to the cell surface. The passage of the adherent clone shows that once *E. coli* DH5α is in close proximity of the epithelial cells this bacterium is also able to pass.

**Restriction endonuclease mapping of clones 9.1 and 9.8.** The 5' and 3' ends of the fragments in clones 9.1 and 9.8 were sequenced and the sequences were compared to the Genbank database using the BLAST Algorithm. The sequences were found to be homologous to sequences of *H*. *influenzae* Rd, of which the whole genome was sequenced (Fig. 5). The DNA sequences obtained with primer PGJB-P1 on clone 9.1 and the sequence obtained with primer PGJB-P2 on clone 9.8 were identical and matched with sequences of ORF HI0642 of the Rd genome sequence. The sequence obtained with primer PGJB-P2 on clone 9.1 was homologous to 100 bp upstream of HI0636 and contained a CCAA repeat similar as found in the Rd genome. The sequence obtained with primer PGJB-P1 on clone 9.8 was homologous to the sequence of HI0637. The length of the cloned fragments corresponded to the size predicted by the Rd genome sequence, suggesting a comparable genetic organization. This implicated that clone 9.1 contained 6 complete ORFs, HI0636 up to HI0641, and clone 9.8 contained 4 complete ORF's (HI0638 up to HI0641) in the reversed orientation compared to that of clone 9.1 (Fig.5).

**Cloning and sequencing of the upstream sequence of ORF HI0636.** To identify the sequences upstream of ORF HI0636, a PCR was designed using primers prMS0637 together with prRDHI0635P1 or prRDHI0635P2 which were based on the sequence of HI0635 of *H*. *influenzae* strain Rd. PCR reactions using these primers on chromosomal DNA of strain A960053 gave no product. In contrast, when performed on chromosomal DNA of *H*. *influenzae* strain Rd and two other nontypeable *H*. *influenzae* strains, products of the expected lengths were amplified (data not shown). This indicated that the genetic organization of strain A960053 upstream of ORF HI0636 differed from that of the other *H*. *influenzae* strains. To obtain upstream sequences, we performed an inside out PCR with primers pr1-0636 and pr2-0636 on completely digested and re-ligated chromosomal DNA of strain A960053. Unexpectedly, this PCR gave a 2 kb product, irrespective of the enzyme we used to cut the DNA. A control experiment pointed out that primer pr2-0636BAM alone also produced this product. The 2 kb product was purified, ligated into the TA vector and transformed to *E*. *coli* DH5α. Sequencing showed that the upstream region of clone 9.1 was highly homologous to *hhuA,* encoding the *H. influenzae* hemoglobin-haptoglobin binding protein, which is not present in Rd.

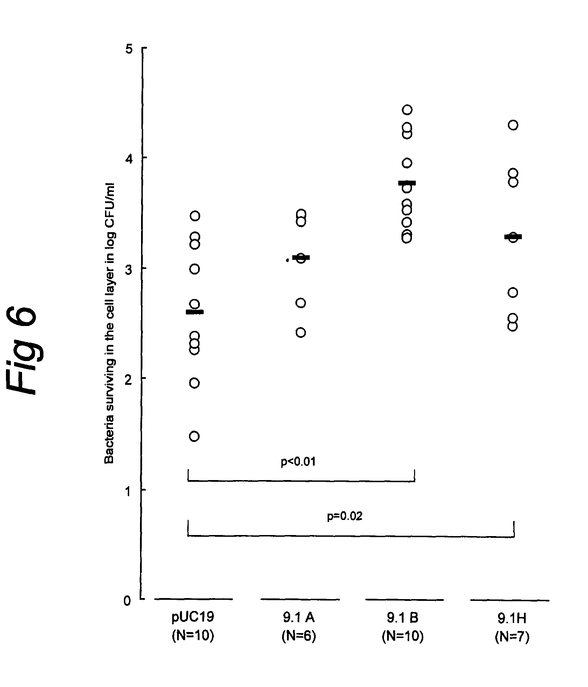

**Characteristics of *E*. *coli* subclones containing ORF's HI0636, HI0637 and HI0638 from *H. influenzae* strain A960053.** Since ORF's HI0637, HI0639, HI0640 and HI0641 had assigned functions which identified them as 'housekeeping genes' (Table 3), they were likely not to be involved in paracytosis. To determine whether ORF's HI0636 and HI0638 that encoded proteins of unknown functions were involved in penetration into the cell layer, several subclones were constructed (Fig. 5). Since ORFs HI0636 and HI0637 have the same orientation and the distance between these ORFs was only 22 bp, they possibly form one operon and were therefore cloned together in one subclone. Clone 9.1B contained a 3.5 kb HincII-XbaI fragment with the complete ORF's HI0637 and HI0636 ligated in pUC19. Clone 9.1H contained a 1.7 kb PstI-EcoRI fragment with the complete ORF HI0638. Clone 9.1A contained a 1.5 kb PstI-EcoRI fragment with the truncated ORF HI0638. The MIC for gentamicin of the subclones was unchanged compared to DH5α containing pUC19 or pGJB103. Alignment of the sequences from the 5' and 3' ends of the cloned fragments of the subclones with the Rd genome sequence confirmed the presence of the expected ORF's. In the paracytosis assay, clones 9.1B and 9.1H passed into the cell layer with higher numbers than the negative control (p<0.01 and p=0.02 respectively) (Fig. 6). These results indicated that both ORF HI0638 and HI0636 but also HI0637 may be involved in penetration of the epithelial cell layers.

| **TABLE 3.** Outline of the 8 open reading frames (ORFs) located on clone 9.1 with their assigned functions. | | |
|---|---|---|
| ORF | Length (aa) | Assigned function |
| *hhuA'* | (953) | hemoglobin binding protein (truncated) |
| HI0636 | 96 | unknown |
| HI0637 | 334 | tryptophanyl-tRNA synthetase (trpS) |
| HI0638 | 205 | unknown |
| HI0639 | 456 | adenylosuccinate lyase (purB) |
| HI0640 | 163 | ribosomal protein L10 (rpL10) |
| HI0641 | 123 | ribosomal protein L7/L12 (rpL7/L12) |
| HI0642' | (456) | UDP-N-acetylglucosamine pyrophosphorylase (truncated) |

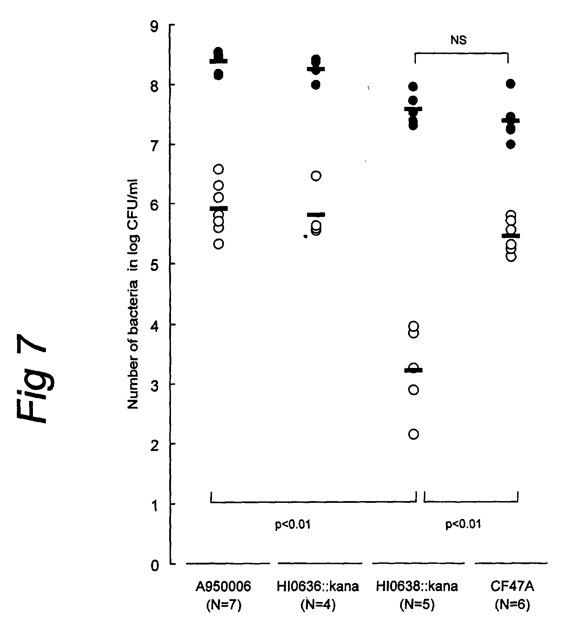

**Paracytosis of *H. influenzae* strain A960053 HI0636 and HI0638 knockout mutants.** To determine the contribution of the ORF's HI0636 and HI0638 to paracytosis in a *H*. *influenzae* background, HI0636 and HI0638 knockout mutants of *H*. *influenzae* strain A960053 were constructed. The numbers of bacteria of *H*. *influenzae* strain A960053 and its HI0636-knock out mutant penetrating into the cell layer were similar (Fig. 7). In contrast, the HI0638-knock out mutant did not penetrate into the cell layer since a 100-fold lower number of bacteria was cultured from the cell layer (Fig. 7). The susceptibility for gentamicin of the HI0638-knock out mutant as determined by E-test was similar to that of the parent strain. However, the HI0638-knock out mutant showed a slightly reduced growth rate compared to the wild type strain A960053. Therefore, the survival of the HI0638 knock out mutant was compared with the survival of *H*. *influenzae* strain CF47A, a slowly growing CF isolate. *H*. *influenzae* strain CF47A as well as the A960053 HI0638 knock out mutant did not grow in the apical medium during the assay, but CF47A penetrated the cell layer in similar numbers as *H*. *influenzae* A960053 (Fig. 7), indicating that the reduced growth rate did cause reduced penetration of the mutant in the cell layer.

Analysis of light microscopy sections of the cell layers 24 h after infection with the *H*. *influenzae* strain A960053 HI0638-knock out mutant showed that indeed only very few bacteria were present in the cell layer compared to the number of bacteria present in the cell layer after infection with the parent strain (data not shown).

### D. Discussion

Hereinabove, the cloning of bacterial genes involved in paracytosis of *H. influenzae* through lung epithelial cell layers is described. Chromosomal libraries were constructed of adherent and a non-adherent nontypeable *H. influenzae* strain in *E. coli* DH5α. An initial attempt, using the library of the adherent *H. influenzae* strain resulted in selection for adherent *E. coli* clones. In the art, it was alreadt shown that adherence to the epithelial cells in vitro leads to elevated penetration rates but that eleven *H. influenzae* isolates tested all penetrated into the cell layers irrespective of their adherence characteristic (van Schilfgaarde, M., P. et al. 1999 Microb Pathog 26:249-262; van Schilfgaarde, M., L. et al. 1995 Infect.Immun. 63:4729-4737). The fact that an adhering clone was isolated indicates that adherence enhances paracytosis, although it is not a prerequisite. Since adherence to the epithelial cell surface concentrates the bacteria at the cell surface, this is not unexpected.

Screening of a chromosomal library of the non-adherent *H. influenzae* strain A960053 in *E. coli* DH5α resulted in the isolation of two clones with enhanced paracytosis, referred to as clones 9.1 and 9.8. Alignment of DNA sequences obtained from these clones showed that the fragments cloned in clones 9.1 and 9.8 were overlapping and homologous to a region present in *H. influenzae* Rd (Fleischmann, R.D. et al., 1995 Science 269:496-512). This was not unexpected since strain Rd also shows paracytosis. Based upon the genome sequence of Rd, the length of different PCR products, and restriction endonuclease patterns, clone 9.1 was concluded to contain 6 complete and 2 truncated ORF's.

Of the 6 complete ORF's two (HI0636 and HI0638) encode small proteins of unknown function. The other ORF's encoded for ribosomal proteins (HI0640 and HI0641), for tryptophanyl-tRNA synthetase (HI0637, *trpS)* and adenylosuccinate lyase (HI0639, *purB*). Of these genes *purB* of *Salmonella* spp. is known to be associated with reduced virulence in mice. However, this is probably due to an effect of this mutation on the survival of the bacterium and not to a specific function in virulence such as interaction with the epithelial cells during infections. Therefore, the role of ORF's HI0636 and HI0638 in the paracytosis model was further investigated. Construction of subclones containing HI0636 or HI0638 and sequencing of these fragments showed that clone 9.1 indeed contained these ORFs similar to strain Rd. However, the genome organization of strain A960053 upstream of ORF HI0636 differed from that of strain Rd. Sequencing of the region upstream of HI0636 of A960053 and data-base searching showed that HI0635 of strain A960053 is homologous to the *hhuA* gene, coding for hemoglobin-haptoglobin binding protein. This gene is absent in strain Rd. Although ORF HI0635 of Rd also seems to encode a hemoglobin binding protein, there is only poor homology (43%) to *hhuA.*

Since ORF HI0638 was present in clone 9.1 and clone 9.8, and these clones both showed an increased survival in the cell layers, this gene was the most likely candidate to encode a paracytin. However, of clone 9.1 higher numbers of bacteria were cultured from in the cell layer than of clone 9.8, indicating that additional sequences in clone 9.1 may influence the paracytosis efficiency. Results of paracytosis assays with two *E. coli* subclones containing either ORF HI0638 or ORF's HI0636 and HI0637 confirmed that these ORF's were indeed involved in increased penetration of the *E. coli* clones. The subclone containing ORF's HI0636 and HI0637 showed higher numbers of bacteria in the cell layer than the clone containing HI0638. The combined results of these experiments indicated a role in penetration for HI0636 as well as HI0638.

Knock out mutation of HI0636 did not result in lower numbers of bacteria in the cell layers, indicating that HI0637 may be important for the interaction with the cell layer as well despite its functionsl assignment as *trpS.* On the other hand, *H*. *influenzae* Rd contains another ORF (HI0400) showing 61% homology to ORF HI0636 when the deduced amino acid sequences are compared. It is possible that HI0400 has the same function as HI0636 and has taken over its function in the HI0636 knockout mutant. Knock out mutation of HI0638 significantly reduced the penetration of the cell layer since a 100 fold lower number of bacteria was cultured from the cell layer. Because HI0638 and HI0639 are located in one operon, an indirect effect of the HI0638 knock out mutation on expression of HI0639 cannot be excluded. However, since HI0639 was not required for paracytosis of *E*. *coli* clones, it can be excluded as the paracytin, and leaves HI0638 as the candidate for a paracytin.

The predicted amino acid sequence of ORF HI0638 is homologous to the YcfC of *E. coli*, a 22.9 kDa membrane associated protein of unknown function. When ORF HI0636 has an auxiliary function related to HI0638 in *H*. *influenzae* it may also act on YcfC in *E. coli,* explaining the effect on paracytosis in the subclone containing only HI0636. Expression of HI0638 also led to an increase in paracytosis, probably because of overexpression of HI0638 from the plasmid compared to the normal level of expression of YcfC.

The passage between the epithelial cells, a characteristic for *H. influenzae*, seems to involve selective disclosure of intercellular junctions (van Schilfgaarde, M., L. et al. 1995 Infect.Immun. 63:4729-4737). The mechanisms by which *H*. *influenzae* is able to disclosure of these junctions are unknown. Other bacteria that employ paracytosis or interjuntional passage are e.g. the spirochetes *Borrelia burgdorferi* and *Treponema pallidum* (Haake, D.A. and M.A. Lovett. 1994 Methods Enzymol 236:447-463). The mechanisms underlying this passage are also unknown. Various intestinal pathogens perturb the paracellular barrier by production of toxins, such as the zonula occludens toxin (ZOT) and the hemagglutin/protease of *Vibrio cholerae*, toxin A and toxin B of *Clostridium difficile*, and VacA of *Helicobacter pylori.* The increase in permeability of the epithelial cell layrer induced by these toxins mostly coincides with a decrease in transepithelial resistance and with a rearrangement of (F-)actin. However, each of the toxins exert different effect the paracellular barrier. VacA increases paracellular epithelial permeability only to low-molecular-mass (<350-440) molecules, without an effect on the junctional proteins that maintain the structure of the intercellular junctions. ZOT has been shown to alter the structure of intercellular junctions apparently by triggering an intracellular cascade which involves protein kinase C. As a consequence of ZOT induced modification of the transepithelial permeability, the intestinal mucosa becomes permeable to water and electrolytes, resulting in diarrhea but not in passage of bacteria through the tight junctions. Toxins A and B of *Clostidium difficile* enhance paracellular transmigration of different bacterial species through cell layers of an intestinal epithelial cell line, indicating a major change in permeability. However these toxins exert a pathologic effect on epithelial cells, which was never observed during passage of *H*. *influenzae* between the epithelial cells.

By which mechanisms *H*. *influenzae* may influence the intercellular permeability of epithelial cells in a subtle, reversible way which permits whole bacteria to protrude is currently unknown. The mechanisms may resemble the mechanism by which leukocytes penetrate through epithelial cell layers. Leukocytes penetrate the cell layers after upregulation of the intercellular adhesion molecule ICAM1 by TNFα. In addition, TNFα affects the tight junctional region between epithelial cells. The increased paracellular permeability of brain endothelial cells induced by TNFα increased virus penetration of HIV by the paracellular route. After infection with *Mycobacterium tuberculosis* an increased permeability of the epithelial cell layer was associated with the production of TNFα by the epithelial cells. Interaction of *H*. *influenzae* or *H*. *influenzae* LPS with different lung epithelial cell lines also induces the production of TNFα as well as other inflammatory mediators such as IL6 and IL8 by the epithelial cells and the expression of ICAM1. However, the transepithelial permeability was either unaltered in our model, or decreased in the study using HBEC lung epitehelial cells, and it is unknown whether *H*. *influenzae* can interact with the ICAM1 molecules.

In conclusion, in the present invention, the paracytin gene of *H*. *influenzae* involved in paracytosis through lung epithelial cells layers was identified and provided. This *inter alia* allows the characterization the paracytin and how it regulates the intercellular permeability. Knowledge of the modification of the function of the intercellular junctions by the *H*. *influenzae* paracytin may enlarge our understanding or the normal physiologic regulation of intercellular junctions and how normal intercellular permeability is regulated.

## Claims

1. Isolated nucleic acid encoding a protein or polypeptide that can be used to alter, and in particular increase, the permeability of a tissue or a cell layer, and in particular of an epithelial cell layer such as a lung epithelial cell layer, said nucleic acid having:
- the nucleotide sequence of SEQ ID NO 1 (ORF HI 0636) and/or SEQ ID NO 2 (ORF HI 0638); and/or
- the nucleotide sequence of a mutant, variant, analogue, part and/or fragment of the nucleotide sequence of SEQ ID NO 1 and/or SEQ ID NO 2; and/or
- the nucleotide sequence of a natural homologue of the nucleotide sequence of SEQ ID NO 1 and/or SEQ ID NO 2; and/or
- the nucleotide sequence of a mutant, variant, analogue, part and/or fragment of a natural homologue of the nucleotide sequence of SEQ ID NO 1 and/or SEQ ID NO 2.

2. Isolated nucleic acid according to claim 1, having:
- the nucleotide sequence of SEQ ID NO 1 (ORF HI 0636) and/or SEQ ID NO 2 (ORF HI 0638); and/or
- the nucleotide sequence of a mutant, variant, analogue, part and/or fragment of the nucleotide sequence of SEQ ID NO 1 and/or SEQ ID NO 2.

3. Isolated nucleic acid according to claim 1 and/or 2, not having the nucleotide sequence of SEQ ID NO 5 and/or SEQ ID NO 6.

4. Protein or polypeptide, encoded by an isolated nucleic acid of any of claims 1-3.

5. Protein or polypeptide according to claim 3, having:
- the amino acid sequence of SEQ ID NO 3 (HI0636) and/or SEQ ID NO 4 (HI 0638); and/or
- the amino acid sequence of a mutant, variant, analogue, part and/or fragment of the amino acid sequence of SEQ ID NO 3 and/or SEQ ID NO 4; and/or
- the amino acid sequence of a natural analog of the amino acid sequence of SEQ ID NO 3 and/or SEQ ID NO 4; and/or
- the amino acid sequence of a mutant, variant, analogue, part and/or fragment of a natural analog of the amino acid sequence of SEQ ID NO 3 and/or SEQ ID NO 4.

6. Protein or polypeptide according to claim 4 or 5, having:
- the amino acid sequence of SEQ ID NO 3 (HI0636) and/or SEQ ID NO 4 (HI 0638); and/or
- the amino acid sequence of a mutant, variant, analogue, part and/or fragment of the amino acid sequence of SEQ ID NO 3 and/or SEQ ID NO 4.

7. Protein or polypeptide according to any of claims 4-6, not having the amino acid sequence encoded by the nucleotide sequence of SEQ ID NO 5 and/or SEQ ID NO 6.

8. Isolated nucleic acid according to any of claims 1-3, and/or protein or polypeptide according to any of claims 4-7, derived from *Haemophilus influenzae.*

9. Isolated nucleic acid according to claim 7, and/or protein or polypeptide according to claim 7, derived from a strain of *Haemophilus influenzae* other than *Haemophilus influenzae* strain Rd.

10. Genetic construct, comprising a nucleic acid according to any of claims 1-3, and optionally one or more further elements of genetic constructs known per se.

11. Use of a protein or polypeptide according to any of claims 5-8 in altering, and in particular in increasing, the permeability of tissues and/or cell layers, and in particular of epithelial cell layers such as lung epithelial cell layers.

12. Use of a protein or polypeptide according to any of claims 5-8 in (the preparation of) a pharmaceutical composition and/or a vaccine.

13. Pharmaceutical composition, at least comprising a polypeptide or protein according to any of claims 5-8, and optionally one or more further therapeutically acive substance, and/or one or more pharmaceutically acceptable carriers, excipientia and/or adjuvans.

14. Vaccine, containing and/or based upon protein or polypeptide according to any of claims 5-8

15. Use of an isolated nucleic acid sequence according to any of claims 1-3, 8 or 9; and/or of a genetic construct according to claim 10, in the transformation of a micro-organism.

16. Micro-organism, transformed with an isolated nucleic acid sequence according to any of claims 1-3, 8 or 9; and/or with a genetic construct according to claim 10.

17. Use of an isolated nucleic acid sequence according to any of claims 1-3, 8 or 9; of a genetic construct according to claim 10; and/or of a micro-organism according to claim 16, in the preparation of a protein or polypeptide according to any of claims 5-8.

18. Vaccine, based upon and/or comprising a micro-organism in which the expression of a nucleotide sequence of SEQ ID NO 1 and/or SEQ ID NO 2 and/or of a natural homologue thereof, and/or the expression of a protein or polypeptide of SEQ ID NO and/or SEQ ID NO 4 or a natural analog thereof, has been reduced and/or essentially fully inhibited.


SEQUENCE LISTING

<110> De Staat der Nederlanden

<120> Proteins and polypeptides from Haemophilus influenzae
      involved in paracytosis, nucleotide sequences encoding
      such proteins or polypeptides, and uses thereof.

<130> BO 43238

<140> EP (first filing)
<141> 2000-03-22

<160> 20

<170> PatentIn Ver. 2.1

<210> 1
<211> 291
<212> DNA
<213> Haemophilus influenzae

<400> 1
atggcaattc aaactgaaaa accgattgaa tgtgtaggtt gtaacacatt tgatatgaaa 60
tcacttttcg ataatcgtga ttgtagtcag gttattgaat atatttatga cggtgaagaa 120
caagtgcaag aagcgctcgc attttttacc cagaaagcgc gtgatgtaga aagtgaacct 180
tgtgaaattc aatctgaaat tacaaaagtg gatgatggtt atttattaaa ggcaaatttc 240
actttttgtt gccaagctga attggtgatt tttcaaatgc gtattgcgta a 291

<210> 2
<211> 618
<212> DNA
<213> Haemophilus influenzae

<400> 2
atgaaaaact accacgatat tgttcttgcc cttgctggcg tatgccaatc agcaaaatta 60
gttcatcaac ttgcaacaga aagtcgagca gattcagata catttttaac cgcacttaat 120
agccttttca ttacccaacc acaacaaacc gaagatgttt ttggtggaga agttcgtcat 180
ttaaaattag gtctagaaac cttaattcat cagctcaatg cacaaggcga tcaaaattta 240
actcgctatt ggctaagcct tttggcatta gaagggaaat tatcgaaaaa tcccgatgcc 300
aaacaaacat taggcaatcg aatttctcgc ctaaaagaac aggaaattca ttacgctcgc 360
gatagtgaaa caatgctatc tattatggca aatatttaca gcgacgttat tagcccattg 420
ggcaaaaaaa ttcacatact aggctcgcct gattatttgc gacaagaact cgtgcaaaat 480
aaaatccgtg cagttttact tgcgggaatt cgatctgccg tgctgtggaa acaaatgggc 540
ggaacaaagt ggcaaatttt atttttccgc agaaaattac ttgccaccgc aaaacagatt 600
tattcttcta tttattaa 618

<210> 3
<211> 96
<212> PRT
<213> Haemophilus influenzae

<400> 3
Met Ala Ile Gln Thr Glu Lys Pro Ile Glu Cys Val Gly Cys Asn Thr
  1               5                  10                  15

Phe Asp Met Lys Ser Leu Phe Asp Asn Arg Asp Cys Ser Gln Val Ile
             20                  25                  30

Glu Tyr Ile Tyr Asp Gly Glu Glu Gln Val Gln Glu Ala Leu Ala Phe
            35                  40                  45

Phe Thr Gln Lys Ala Arg Asp Val Glu Ser Glu Pro Cys Glu Ile Gln
        50                  55                  60

Ser Glu Ile Thr Lys Val Asp Asp Gly Tyr Leu Leu Lys Ala Asn Phe
65                  70                  75                  80

Thr Phe Cys Cys Gln Ala Glu Leu Val Ile Phe Gln Met Arg Ile Ala
                85                  90                  95

<210> 4
<211> 205
<212> PRT
<213> Haemophilus influenzae

<400> 4
Met Lys Asn Tyr His Asp Ile Val Leu Ala Leu Ala Gly Val Cys Gln
1               5                   10                  15

Ser Ala Lys Leu Val His Gln Leu Ala Thr Glu Ser Arg Ala Asp Ser
            20                  25                  30

Asp Thr Phe Leu Thr Ala Leu Asn Ser Leu Phe Ile Thr Gln Pro Gln
        35                  40                  45

Gln Thr Glu Asp Val Phe Gly Gly Glu Val Arg His Leu Lys Leu Gly
    50                  55                  60

Leu Glu Thr Leu Ile His Gln Leu Asn Ala Gln Gly Asp Gln Asn Leu
65                  70                  75                  80

Thr Arg Tyr Trp Leu Ser Leu Leu Ala Leu Glu Gly Lys Leu Ser Lys
                85                  90                  95

Asn Pro Asp Ala Lys Gln Thr Leu Gly Asn Arg Ile Ser Arg Leu Lys
            100                 105                 110

Glu Gln Glu Ile His Tyr Ala Arg Asp Ser Glu Thr Met Leu Ser Ile
        115                 120                 125

Met Ala Asn Ile Tyr Ser Asp Val Ile Ser Pro Leu Gly Lys Lys Ile
    130                 135                 140

His Ile Leu Gly Ser Pro Asp Tyr Leu Arg Gln Glu Leu Val Gln Asn
145                 150                 155                 160

Lys Ile Arg Ala Val Leu Leu Ala Gly Ile Arg Ser Ala Val Leu Trp
                165                 170                 175

Lys Gln Met Gly Gly Thr Lys Trp Gln Ile Leu Phe Phe Arg Arg Lys
            180                 185                 190

Leu Leu Ala Thr Ala Lys Gln Ile Tyr Ser Ser Ile Tyr
        195                 200                 205

<210> 5
<211> 291
<212> DNA
<213> Haemophilus influenzae

<400> 5
atggcaattc aaaccgaaaa accgattgaa tgtgtaggtt gtaatacttt tgatatgaaa 60
tcacttttcg ataatcgtga ttgtagtcag gttattgaat atatttatga cagtgaagga 120
caagcgcaag aagcactcgc attttttact cagaaagcac gtgatgtaga aagtgaacct 180
tgtgaaattc aatctgaaat tacaaaagtg gatgatggct atttattaaa ggcagatttc 240
actttctgtt gccaagctga attggtgatt tttcaaatgc gtattgcata a 291

<210> 6
<211> 618
<212> DNA
<213> Haemophilus influenzae

<400> 6
atgaaaaact accacgatat tgttcttgcc cttgctggcg tgtgccaatc agcaaaattg 60
gttcatcaac ttgcaacaga aagtcgagca gattcagaga catttttaac cgcacttaat 120
agccttttca ttacccagcc tcaacgaatt gaagatgttt ttggtggcga agttcgtcat 180
ttaaaattag gtctagaaac cttaattcat cagctcaacg cgcaaggcga tcaaaattta 240
actcgctatt ggctaagcct tttagcatta gaaggaaaat tatcgaaaaa ttccgatgcc 300
aaacaaacat tgggcaatcg aatttctcgc ctaaaagaac aagaaattca ttacgctcgt 360
gatagtgaaa caatgctatc tattatggca aatatttaca gcgatattat tagcccattg 420
ggcaaaaaaa ttcacatact aggctcgcct gattatttgc gacaagaact cgtgcaaaat 480
aaaatccgtg cagttttact tgcaggcatt cgctctgccg tgctgtggaa acaaatgggc 540
ggaacaaaat ggcaaatttt atttttccgc agaaaattac ttgccaccgc aaaacagatt 600
tattcttcta tttattaa 618

<210> 7
<211> 21
<212> DNA
<213> Artificial Sequence

<220>
<223> Description of Artificial Sequence: primer

<400> 7
gcttgtcctt cactgtcata a 21

<210> 8
<211> 21
<212> DNA
<213> Artificial Sequence

<220>
<223> Description of Artificial Sequence: primer

<400> 8
gcaagaagca ctcgcatttt t 21

<210> 9
<211> 20

<212> DNA
<213> Artificial Sequence

<220>
<223> Description of Artificial Sequence: primer

<400> 9
ctgctcgact ttctgttgca                                                    20


<210> 10
<211> 21
<212> DNA
<213> Artificial Sequence

<220>
<223> Description of Artificial Sequence: primer

<400> 10
ccaaccacaa caaaccgaag a                                                  21


<210> 11
<211> 23
<212> DNA
<213> Artificial Sequence

<220>
<223> Description of Artificial Sequence: primer

<400> 11
gtggctttag cccaccaaca aac                                                23


<210> 12
<211> 27
<212> DNA
<213> Artificial Sequence

<220>
<223> Description of Artificial Sequence: primer

<400> 12
ctcacgttcc tgaacacacg caattaa                                            27


<210> 13
<211> 26
<212> DNA
<213> Artificial Sequence

<220>
<223> Description of Artificial Sequence: primer

<400> 13
gatagttaat tcaccagaag gctgca                                             26


<210> 14
<211> 25
<212> DNA

<213> Artificial Sequence

<220>
<223> Description of Artificial Sequence: primer

<400> 14
gtgcgtagtg gcgttacttt atctt                                          25


<210> 15
<211> 28
<212> DNA
<213> Artificial Sequence

<220>
<223> Description of Artificial Sequence: primer

<400> 15
gaacgtcctg ctaatgtaag tgtcttaa                                       28


<210> 16
<211> 26
<212> DNA
<213> Artificial Sequence

<220>
<223> Description of Artificial Sequence: primer

<400> 16
caacccctcg aactgcataa ccactt                                         26


<210> 17
<211> 25
<212> DNA
<213> Artificial Sequence

<220>
<223> Description of Artificial Sequence: primer

<400> 17
ccgctcatga gacaataacc ctgat                                          25


<210> 18
<211> 26
<212> DNA
<213> Artificial Sequence

<220>
<223> Description of Artificial Sequence: primer

<400> 18
gggaataagg gcgacacgga aatgtt                                         26


<210> 19
<211> 25
<212> DNA
<213> Artificial Sequence

<220>
<223> Description of Artificial Sequence: primer

<400> 19
cgttgtaaaa cgacggccag tgaat                                          25


<210> 20
<211> 25
<212> DNA
<213> Artificial Sequence

<220>
<223> Description of Artificial Sequence: primer

<400> 20
atttcacaca ggaaacagct atgac                                          25